# EUROPÄISCHE PATENTANMELDUNG

(11) **EP 1 356 818 A2**
(43) Veröffentlichungstag der Anmeldung: **29.10.2003**
(21) Anmeldenummer: 03017188.8
(22) Anmeldetag: 04.11.1996
(51) Int. Cl.: A61K 38/17, A61K 39/39, A61K 48/00, A61P 37/02

(54) **Medikament, insbesondere zur Modulation der Immunantwort bei der Bekämpfung von Viren, Tumoren, Bakterien und Parasiten**

(30) Priorität: 06.11.1995 DE 19541284
(62) Teilanmeldung aus: 96938062.5
(71) Anmelder: Kalden, Joachim Robert, 91054 Erlangen (DE)
(72) Erfinder: Die Erfindernennung liegt noch nicht vor
(74) Vertreter: Gassner, Wolfgang

(57) **Zusammenfassung**

Die Erfindung betrifft ein Medikament, insbesondere zur Modulation der Immunantwort bei der Bekämpfung von Viren, Tumoren, Bakterien und Parasiten, wobei zur Beeinflussung der phosphatidlyserinabhängigen Phagozytose ein aus der folgenden Gruppe ausgewählter Wirkstoff enthalten ist: Annexin, Annexin-Antikörper, Annexin-Liganden oder Phosphatidlyserin.

## Beschreibung

Die Erfindung betrifft ein Medikament, insbesondere zur Modulation der Immunantwort bei der Bekämpfung von Viren, Tumoren, Bakterien und Parasiten. Die Erfindung betrifft ferner die Verwendung eines Wirkstoffs zur Herstellung eines Medikaments.

Phosphatidylserin ist ein negativ geladenes Phospholipid, das sich bei allen Zellen an der Innenseite der Zytoplasmamembran befindet. Gelegentlich kann jedoch ein Phosphatidylserinmolekül durch die Membran schwingen und somit auf die Außenseite der Zytoplasmamembran gelangen. Bei lebenden gesunden Zellen wird auf die Außenseite gelangtes Phosphatidylserin sofort enzymatisch auf die Innenseite der Zytoplasmamembran zurücktransportiert. Bei alten und bei Plasmodium faiciparum infizierten Erythrozyten, bei Sichelzellen, postinflammatorischen Granulozyten sowie bei apoptotischen Zellen bleibt das Phosphatidylserin jedoch auf der Außenseite. Ab einer bestimmten Phosphatidylserindichte binden die Zellen über den "Phosphatidylserinrezeptor" an Phagozyten. Steigt die Phosphatidylserindichte weiter an und erreicht dabei einen bestimmten Schwellenwert, werden die Zellen extrem schnell phagozytiert (Engulfmentphagozytose). Bei diesem Vorgang kommt es weder zum Freisetzen von Zellinhaltsstoffen noch zu einer Aktivierung des Immunsystems. Aus diesem Grund wird dieser Phagozytoseweg "nichtinflammatorisch" genannt.

Bei der Beseitigung gealterter Zellen, z. B. alter Erythrozyten und apoptotischer Zellen, wie postinflammatorischer Granulozyten, ist eine spezifische Immunsupression durchaus sinnvoll und erwünscht, da in diesen Fällen eine inflammatorische Phagozytose sogar zu Autoimmunphänomenen führen könnte. Die nichtinflammatorische Phagozytose von Plasmodium falciparum infizierten Erythrozyten ist jedoch unter anderem für die ausgesprochen schlechte Immunantwort und die schwierige Immunisierung gegen Malaria verantwortlich. Keine bisher beschriebene Maßnahme oder Vorbeugung gegen Malaria berücksichtigt den Umstand, daß Plasmodium falciparum infizierte Erythrozyten über den phosphatidylserinabhängigen Engulfmentphagozytoseweg phagozytiert werden. - Medikamente, die diesen Phagozytoseweg beeinflussen, sind bisher unbekannt.

Ähnlich verhält es sich bei Virusinfektionen. Viren, die über phosphatidylserinabhängige Phagozytose aus apoptotischen Zellen in Phagozyten aufgenommen werden, können so der Immunüberwachung entgehen. So ist z. B. die Aufnahme von HIV in Monozyten, die ohne Auslösung des "respiratory bursts" vonstatten geht, für das frühe und vom Immunsystem unbemerkte Eindringen des HIV in den langlebigen Monozyten-Pool verantwortlich. Diese bisher unverstandene Infektion der Monozyten/Makrophagen wird für die Persistenz des HIV und somit für die Ausbildung des AIDS-Krankheitsbilds ursächlich verantwortlich gemacht. Obwohl der Infektionsweg von Monozyten/Makrophagen mit HIV bisher molekular noch nicht eindeutig identifiziert ist, ist eine Beteiligung von Phosphatidylserin und Phosphatidylserinrezeptor wegen der nichtinflammatorischen Phagozytose wahrscheinlich. So konnte z. B. gezeigt werden, daß Retrovirusgenome aus apoptotischem Debris in Zellen aufgenommen werden können und so diese Zellen infizieren. Da HIV in den Monozyten sehr lange überleben kann und, eventuell erst Jahre nach der Infektion, spontan freigesetzt wird, kann das menschliche Immunsystem das HIV nicht völlig aus dem Körper eliminieren. Da das HIV bei jeder Freisetzung das Immunsystem etwas schädigt, indem es die CD 4 positiven T-Zellen zerstört, kann sich so im Laufe meist mehrerer Jahre das Vollbild des AIDS ausbilden. Ähnliche Probleme existieren auch bei der Eliminierung von anderen in Phagozyten persistierenden bzw. sich vermehrenden Viren. Hier sind vor allem weitere Retroviren und besonders die Untergruppe der Lentiviren zu nennen. Einige dieser Viren (EIAV, Meadi Visna Virus, CAEV) persistieren in den Phagozyten von Huftieren und führen dort zu Autoimmnunkrankheiten. Keine bisher beschriebene Maßnahme oder Vorbeugung gegen HIV Infektion bzw. der Infektion mit anderen in Phagozyten überlebenden Viren berücksichtigt den Umstand, daß apoptotische Zellen über den phosphatidylserinabhängigen Weg phagozytiert werden können. - Medikamente, die diesen Phagozytoseweg blockieren, sind bisher unbekannt.

Anders stellt sich die Situation bei Patienten mit Sichelzellanämie dar. Durch die ständige und extrem schnelle Phagozytose autologer, genetisch veränderter Erythrozyten kommt es bei den Patienten zu einer Anämie, die unbehandelt in schweren Fällen zum Tod führen kann. Hier steht weniger die Tatsache im Vordergrund, daß die phosphatidylserinvermittelte Phagozytose nichtinflammatorisch verläuft, als vielmehr der Umstand, daß sie extrem schnell und effizient Phosphatidylserin tragende Zellen beseitigt. - Da es bisher keine Medikamente gibt, die diesen Phagozytoseweg blockieren, wird die Sichelzellanämie bisher mit wiederholten Bluttransfusionen behandelt.

Ein ähnliches Problem wie bei der Sichelzellanämie ergibt sich auch beim Lagern von Erythrozyten zur Transfusion. Auch bei Lagerung unter Blutbankbedingungen exprimiert eine zunehmende Anzahl Erythrozyten Phosphatidylserin auf ihrer Oberfläche. Dies führt nach der Transfusion dazu, daß diese Erythrozyten sehr schnell von Phagozyten abgeräumt und so wirkungslos werden. - Da es bisher keine Medikamente bzw. Zusätze zu Blutkonserven gibt, die diese Phagozytose verhindern, ist die Lagerung von Erythrozyten zeitlich strikt limitiert.

Bei der Herstellung von Tumorvakzinen werden die in den Körper der Patienten bzw. Versuchstiere zurückgegebenen Tumorzellen bestrahlt, um eine weitere Verbreitung des Tumors zu verhindern. Da unter diesen Umständen in den Tumorzellen Apoptose induziert wird und diese dann nichtinflammatorisch über den phosphatidylserinabhängigen Engulfmentphagozytoseweg beseitigt werden, kommt es meist zu einer relativ schwachen Immununantwort gegen die jeweilige Tumorvakzine. - Da bisher keine Substanzen bekannt sind, die den phosphatidylserinabhängigen Phagozytoseweg blockieren, werden bisher klassische Immunisierungswege und Adjuvantien verwendet, um die Immunantwort gegen Tumorzellen zu steigern.

Nach dem Stand der Technik sind des weiteren Annexine bekannt. Diese bilden eine hochkonservierte Familie zellulärer Proteine, die von höheren Pflanzen, über Wirbellose, Fische und Vögel bis hin zu den Säugetieren gefunden werden (Nomenklatur: Crumpton MJ and Dedman JR, 1990: "Protein terminology tangle"; Nature 345: 212). Diese zytoplasmamembranassoziierten Proteine besitzen entweder ein niedrigeres (32 kD bis 38 kD) oder aber ein höheres (ca. 67 kD) Molekulargewicht. Sie zeichnen sich außerdem durch eine Affinität zu Ca++ und Phospholipiden aus. Eine hochkonservierte Core-Struktur besteht aus vier oder acht Wiederholungen von je 70 Aminosäuren, die jeweils einen Endonexinbereich mit 17 Aminosäuren enthalten, die an der Ca ++ -Bindung beteiligt sind. In den aminoterminalen Regionen der unterschiedlichen Annexine werden keine signifikanten Ähnlichkeiten gefunden, was zu der Spekulation geführt hat, daß diese Domäne für die unterschiedlichen Funktionen der Annexine verantwortlich ist. Obwohl für Annexine eine Vielzahl von Funktionen postuliert wurden, wie z. B. die Inhibition der Phospholipase A₂ und der Blutgerinnung, sowie eine mögliche Beteiligung an Signaltransduktion, Zeliwachstum und Differenzierung, konnte bisher keine eindeutige biologische Rolle für die Armexine etabliert werden (Morgan MO und Femandez MP, 1991: "Annexins and signal transduction" in Bailey JM (ed): "Advances in Prostaglandine, Leukotrien, Lipoxin and PAF Research"; New York: Plenum Press: 107-122).

Die Röntgenstrukturanalyse der Annexine vom Typ V aus Mensch, Huhn und Ratte ergab, daß Annexin V ein kanalbildendes integrales Membranprotein ist (Huber R, Römisch J, Paques E, 1990: "The crystal and molecular structure of human Annexin V, an anticoagulant protein that binds to calcium and membranes"; The EMBO Journal 9: 3867-3874). Das Protein wird in Zytoplasma und Kern gefunden. Obwohl es nur partiell in der Innenseite in die Zytoplaamamembran integriert ist, konnte eine spannungsabhängige Kalziumkanalaktivität nachgewiesen werden. Außerdem wird Annexin V auch auf der Oberfläche von Chondrozyten gefunden, wo es an der Knorpelkalzifizerung beteiligt ist (Pfäffle M, Ruggiero F, Hofmann H, Femandez MP, Seimin 0, Yamada Y, Garrone R und von der Mark K, 1988: "Biosynthesis, secretion and extracellular locafization of anchorin CII, a collagen-binding protein of the calpactin family"; The EMBO Journal 7/8, 2335-2342).

Die nach dem Stand der Technik bekannten Medikamente und Verfahren, insbesondere die heutzutage eingesetzten Adjuvantien stimulieren das Immunsystem unspezifisch. Bisher ist noch kein Agens beschrieben, das insbesondere die Engulfmentphagozytose von Phosphatidylaerin tragenden Zellen verhindert und so zu einer spezifischen Immunstimulation führt. Besonders hervorzuheben sind die Nachteile, die bei nichtinflammatorischem Abräumen von Impfstoffen und Viren entstehen. Einerseits führt das zum wirkungslosen Abbau von Impfstoffen, andererseits zur Viruspersistenz.

Da bei der Sichelzellanämie bereits junge Erythrozyten Phosphatidylserin auf ihrer Oberfläche tragen, werden sie von den körpereigenen Phagozyten entfernt. Das trägt nachteiligerweise zur Anämie der Patienten bei.

Auch für die Lagerung von Blut und Erythrozyten sind keine Medikamente bzw. Zusätze bekannt, die den Abbau der Spendererythrozyten durch die Empfängerphagozyten nach der Transfusion verhindern. Das führt bislang zu einer relativ kurzen Lagerungsfähigkeit von Blutkonserven und Erythrozytenkonzentraten bzw. zu einem deutlichen Verlust an Wirksamkeit bei länger gelagerten Konserven.

Aufgabe der vorliegenden Erfindung ist es, die Nachteile nach dem Stand der Technik zu beseitigen. Insbesondere soll ein Medikament bzw. die Verwendung eines Wirkstoffs angegeben werden, welches/r eine Erhöhung der Immunität gegen Viren, Tumoren, Bakterien und Parasiten bewirkt.

Diese Aufgabe wird durch die Merkmale der Patentansprüche 1 und 6 gelöst. Zweckmäßige Weiterbildungen ergeben sich aus den Merkmalen der Patentansprüche 2 bis 5 bzw. 7 bis 10.

Nach Maßgabe der Erfindung ist in einem Medikament, insbesondere zur Modulation der Immunantwort bei der Bekämpfung von Viren, Tumoren, Bakterien und Parasiten, zur Beeinflussung der phospatidylserinabhängigen Phagozytose, ein aus der folgenden Gruppe ausgewählter Wirkstoff enthalten: Annexin, Annexin-Antikörper, Annexin-Liganden/ Phosatidylserin oder Phophodiesderase.

Erfindungsgemäß ist daneben die Verwendung eines Wirkstoffs zur Herstellung eines Medikaments, insbesondere zur Modulation der Immunantwort bei der Bekämpfung von Viren, Tumoren, Bakterien und Parasiten, vorgesehen, wobei der Wirkstoff zur Beeinflussung der phosphatidlyserinabhängigen Phagozytose aus der folgenden Gruppe ausgewählt ist: Annexin, Annexin-Antikörper, Annexin-Liganden, Phosatidylserin oder Phophodiesderase.

Durch die Zugabe eines der erfindungsgemäßen Wirkstoffe wird der Annexinspiegel verändert und/oder die Verteilung von Annexinen verändert. Des weiteren können damit die Wirkorte oder Wirkpartner des Phospatidylserins modifiziert werden.

Durch Annexin, vorzugsweise durch Annexin V, kann die phosphatidylserinabhängige Phagozytose, insbesondere die Engulfmentphagozytose, moduliert oder inhibiert werden. Außerdem kann beispielsweise durch Entfernen bzw. eine Blockade von Annexinen, insbesondere von Annexin V, die Engulfmentphagozytose moduliert oder stimuliert werden.

Besondere Bedeutung ist der Anwendung im human- bzw. tiermedizinischen Bereich zuzumessen, wo in vielen etablierten, aber auch in experimentellen Therapieformen eine Immunmodulation erwünscht ist. So ist in der Behandlung von Tumorerkrankungen und von Virusinfektionen oft eine Immunstimulation erwünscht, wohingegen bei Erkrankungen des rheumatischen Formenkreises und bei Autoimmunerkrankungen eher eine Immunsupression erwünscht ist.

Ein wichtiges Einsatzgebiet für die Blockade der nichtinflammatorischen phosphatidylserinabhängigen Engulfmentphagozytose ergibt sich aus der daraus resultierenden spezifischen "Adjuvanswirkung". Phosphatidylserin tragende Zellen werden nach der Blockade des nichtinflammatorischen phosphatidylserinabhängige Engulfmentphagozytosewegs über einen inflammatorischen immunstimulatorischen Alternativweg phagozytiert, was mit einer massiv erhöhten Immunantwort einhergeht. Für diese "Adjuvanswirkung" ergeben sich die unterschiedlichsten Anwendungsgebiete, z. B. in der Humanmedizin. Zum einen kann dadurch die Immunogenität von Tumorvakzinen gesteigert werden, wenn diese aus bestrahlten und somit größtenteils apoptotischen Tumorzellen bestehen. Weiterhin ist es möglich, eine Immunantwort gegen solche Tumorzellen zu erzielen, die aus therapeutischen Gründen in situ radioaktiv bestrahlt werden. In diesem Fall würde eine tumorspezifische Immunantwort bei der Beseitigung der Resttumormasse den Therapieerfolg steigern. Ein ähnlicher Effekt kann auch parallel zu einer Zytostatikatherapie mit apoptoseinduzierenden Agentien, wie z. B. Cisplatin und Hydroxy-Harnstoff, zu einer massiven tumorspezifischen Immunstimulation führen. Auch bei der Behandlung von Virusinfektionen, z. B. von solchen Viren, die in Phagozyten persistieren, führt die Blockierung des phosphatidylserinabhängigen Engulfmentphagozytosewegs zu einer spezifischen Immunstimulation. Als besonders wichtiges Beispiel in diesem Zusammenhang muß die Behandlung der Infektionen mit Lentiviren und HIV angesehen werden. Ein von der Zelle "unbemerktes" phosphatidylserinabhängiges Eindringen der Viren führt zur Viruspersistenz im langlebigen Monozyten/Makrophagen-Pool. Die Viruspersistenz führt bei den allermeisten Infizierten nach einer mehr oder minder langen Latenzzeit zum Tode. Annexine, bevorzugt Annexin V, sind für die Behandlung von HIV-Infizierten geeignet, da inflaromatorisch phagozytiertes apoptotisches Material in den Phagozyten einen "respiratory burst" auslöst und so zur Zerstörung der Virengenome führen würde.

Weiterhin können durch die Blockierung der phosphatidylserinabhängigen Engulfmentphagozytose unerwünschte Zellverluste in vivo und in vitro vermieden werden. Das ist sowohl bei der Lagerung von erythrozytenhaltigen Blutkonserven als auch als Medikament für Patienten mit Sichelzellanämie von großer Bedeutung.

### Beispiele:

1. Einsatz von Annexinen, bevorzugt Annexin V, als Adjuvantien für Tumorvakzinen
   Zur Produktion von Tumorvakzinen aus von Patienten isolierten Tumorzellen, werden diese vor der Reinjektion in den Patienten radioaktiv bestrahlt, um ein Anwachsen zu verhindern. Während der dadurch induzierten Apoptose wird auf der Oberfläche der Tumorzellen Phosphatidylserin exponiert, was zu einer schwachen Immunogenität der Tumorvakzine führt. Direkt vor der Injektion werden die bestrahlten Tumorzellen ex *corpore* mit Annexinen, bevorzugt Annexin V, inkubiert um die phosphatidylserinabhängige Engulfmentphagozytose im Patienten zu blockieren. Zusätzlich wird an die Stelle der Injektion ein Annexin, bevorzugt Annexin V, Bolus gesetzt, um die Wirkung lokal weiter zu verstärken.
2. Einsatz von Annexinen, bevorzugt Annexin V, als Immunstimulans bei der Chemo- und Strahlentherapie
   Bei der therapeutischen radioaktiven Tumorbestrahlung sowie bei der Behandlung mit Zytostatika wird in corpore in einer Vielzahl von Tumorzellen Apoptose induziert. Um ein nichtinflammatorisches Abräumen der toten Zellen zu verhindern und die damit verbundene schwache Immunantwort zu verstärken, werden vor oder unmittelbar nach der Strahlen- bzw. Chemotherapie Annexine, bevorzugt Annexin V, in den Tumor injiziert. Dadurch erfolgt das Abräumen der toten Tumorzellen über einen inflammatorischen Phagozytoseweg und führt so zu einer verstärkten Immunantwort gegen den Resttumor.
3. Lagerung von Vollblut und Erythrozytenpräparaten
   Zu Vollblut- bzw. Erythrozytenkonzentraten werden Annexine, bevorzugt Annexin V, zugefügt, um nach der Transfusion den Abbau der Phosphatidylserin tragenden Erythrozyten zu verlangsamen und so die Wirksamkeit der Transfusion zu erhöhen. Die Annexine, bevorzugt das Annexin V, können in diesem Fall entweder direkt nach der Blutabnahme oder aber auch erst vor der Transfusion zugegeben werden.
4. Einsatz von Annexinen, bevorzugt Annexin V, bei Patienten mit Sichelzellanämie
4.a Klassische Lösung mit Annexin-, bevorzugt Annexin V-, Infusionen
   Um die Phagozytose der Sichelzellen zu verhindern, die bei diesem Krankheitsbild entscheidend zur Anänmie beiträgt, werden sehr stark anämischen Patienten Annexine, bevorzugt eine Annexin V Lösung, intravenös verabreicht.
4.b Einsatz von Annexinen, bevorzugt Annexin V, im transient gentherapeutischen Ansatz mit RNA-Virus abgeleiteten Vektoren
   In diesem Ansatz wird ein Fusionsprotein aus Annexinen, bevorzugt Annexin V, mit einem Leaderpeptid mit Hilfe eines transienten RNA-Vektorsystems (z. B. einem vom Poliovirus abgeleiteten System) in Blutzellen, z. B. Monozyten, zur Expression gebracht. Auf diese Weise blokkiert diese transiente in situ Produktion über einen längeren Zeitraum als eine Infusion die Phagozytose der Sichelzellen. Da RNA-abhängige Expressionssysteme sich weder in die genomische DNA der Wirtszellen integrieren noch vertikal verbreiten, ist die Expression der Annexine nur transient, was die Risiken der Auslösung einer Autoimmnunopathie minimiert.
5. Auch bei der Behandlung von Infektionen mit Viren, z. B. von solchen, die in Phagozyten persistieren, führt die Blockierung des phosphatidylserinabhängigen Engulfmentphagozytosewegs zu einer spezifischen Immunstimulation. Als besonders wichtiges Beispiel in diesem Zusammenhang muß die Behandlung der Infektionen mit Lentiviren und HIV angesehen werden. Ein von der Zelle "unbemerktes" Eindringen der Viren führt zur Viruspersistenz im langlebigen Monozyten/Makrophagen-Pool und bei den allermeisten Infizierten nach einer mehr oder minder langen Latenzzeit zum Tode. Annexine, bevorzugt Annexin V, sind für die Behandlung von HIV geeignet, da inflammatorisch phagozytiertes apoptotisches Material in den Phagozyten einen "respiratory burst" auslöst und so zur Zerstörung der Virengenome führt.

## Patentansprüche

1. Medikament zur Erhöhung einer Immunantwort enthaltend Annexin vom Typ V.

2. Medikament nach Anspruch 1, wobei die Immunantwort eine Immunantwort zur Bekämpfung von Bakterien ist.

3. Medikament nach Anspruch 1 oder 2, wobei das Annexin vom Typ V Bestandteil eines Fusionsproteins ist.

4. Medikament nach Anspruch 3, wobei das Fusionsprotein mit einem RNA-Vektor gekoppelt ist.

5. Medikament nach Anspruch 4, wobei der RNA-Vektor vom Polio-Virus abgeleitet ist.

6. Verwendung von Annexin vom Typ V zur Herstellung eines die Immunantwort erhöhenden Medikaments.

7. Verwendung nach Anspruch 6, wobei die Immunantwort eine Immunantwort zur Bekämpfung von Bakterien ist.

8. Verwendung nach Anspruch 6 oder 7, wobei das Annexin vom Typ V Bestandteil eines Fusionsproteins ist.

9. Verwendung nach Anspruch 8, wobei das Fusionsprotein mit einem RNA-Vektor gekoppelt ist.

10. Verwendung nach Anspruch 9, wobei der RNA-Vektor Polio-Virus abgeleitet ist.
